# EUROPÄISCHE PATENTSCHRIFT

(11) **EP 0 491 984 B1**
(45) Veröffentlichungstag und Bekanntmachung des Hinweises auf die Patenterteilung: **22.12.1993**
(21) Anmeldenummer: 90125453.2
(22) Anmeldetag: 24.12.1990
(51) Int. Cl.: F16L 47/04, A61M 39/00

(54) **Anordnung zur Verbindung zweier Fluid-Systemabschnitte**
Construction for joining two fluid system sections
Ensemble de raccord pour deux systèmes fluides

(43) Veröffentlichungstag der Anmeldung: 01.07.1992
(73) Patentinhaber: PALL CORPORATION, Glen Cove, New York 11542 (US)
(72) Erfinder: Baumbach, Frank, W-6074 Rödermark (DE); Otto, Ulrich, W-6052 Mühlheim (DE); Randhahn, Horst Dr., W-6100 Darmstadt-Eberstadt (DE)
(74) Vertreter: Altenburg, Udo, Dipl.-Phys.

(56) Entgegenhaltungen:
- WO-A-83/00812
- CH-A- 480 581

## Beschreibung

Die Erfindung betrifft eine Verbindung von zwei Fluid-Systemabschnitten, vorzugsweise eines aseptischen Systems.

Es ist eine Aseptik-Verschraubung mit O-Ring bekannt (RA 2287 der Firrna SÜDMO-Amaturenfabrik GmbH), bei der zwei Rohrabschnitte mittels einer Überwurfmutter verbunden werden. Beide Rohrabschnitte haben an ihren Enden innenumfänglich jeweils eine etwa viertel- bis halbkreisförmige Nut derart, daß im verschraubten Zustand der zwei Rohrabschnitte ein O-Ring so in den durch die zwei Nuten gebildeten Raum eingespannt ist, daß der Innendurchmesser des O-Rings mit den Innendurchmessern der zwei Rohrabschnitte etwa bündig abschließt. Diese Aseptikverschraubung ist für Anwendungen geeignet, bei denen ein Differenzdruck von innen nach außen wirkt, der Druck im Inneren der Rohrverschraubung also größer ist als außerhalb der Rohrverschraubung. Im Dichtungsbereich gibt es bei dieser Aseptikverschraubung keine Spalten oder Hohlräume, in denen sich Bakterien dauerhaft ansiedeln könnten.

Weiterhin ist von der Firma Pall ein Aseptik-Adapter zur Verbindung eines Filterelements mit einem Rohrabschnitt bekannt (Doppel-O-Ring-Adapter Code 7 der Firma Pall), bei dem ein Stutzen des Filterelementes einen Ansatz für eine Bajonettverriegelung sowie am Ende des Stutzens zwei radiale Außennuten hat, die zur Aufnahme von jeweils einem O-Ring geeignet sind. Der an den Stutzen anzuschließende Rohrabschnitt hat ein Bajonettelement sowie einen Abschnitt, der den Öffnungen der zwei Nuten des Stutzens im verriegelten Zustand gegenüberliegt und die beiden O-Ringe komprimiert. Die Konstruktion ist derart, daß der Innendurchmesser des Stutzens und der Innendurchmesser des Rohrabschnitts etwa gleich sind und im verriegelten Zustand zwischen den zwei Elementen nur ein kleiner Spalt verbleibt. Diese Anordnung ist geeignet für Anwendungen, bei denen der Differenzdruck von außen nach innen wirkt, der Druck innerhalb des Adapters also kleiner ist als außerhalb. Diese Anordnung kann insofern kritisiert werden, als daß über den O-Ringen ein Spaltraum liegt, der nicht leer, d.h. trockenlaufen kann, und daß zwischen den beiden O-Ringen ein abgeschlossener Raum entsteht, der nicht vom Sterilisationsdampf berührt wird.

Die PCT Veröffentlichung WO 83-00812, die eine Verbindung mit den Merkmalen des Oberbegriffs von Anspruch 1 zeigt, offenbart ein Kopplungsstück, bei dem die beiden Kopplungsteile durch einen Schnappverschluß miteinander verbunden sind und zusätzlich eine Uberwurfmutter, die beide Teile zusammenhält. Durch zwei O-Ringe, die an beiden Enden der Überwurfmutter angeordnet sind, wird die Übergangsstelle zwischen den beiden Kopplungsteilen nach außen vollkommen dicht abgeschlossen.

Es ist die Aufgabe der Erfindung, eine Anordnung zur Gas- oder Fluid-Verbindung von zwei rohrartigen Systemabschnitten unter Vermeidung von Toträumen anzugeben, wobei der Differenzdruck von außen nach innen wirkt.

Diese Aufgabe wird durch eine Verbindung mit den Merkmalen des Anspruchs 1 gelöst.

Die erfindungsgemäße Adapteranordnung bzw. Verbindung soll vorzugsweise zur Gas- oder Fluid-Verbindung von Systemabschnitten eines aseptischen Systems eingesetzt werden. Zur Gewährleistung einer bakteriensicheren und totraumfreien Dichtung ist es erforderlich, daß der O-Ring immer unter Kompression steht (auch während einer Sterilisation) und Kondensat auf beiden Seiten der Dichtung ablaufen kann. Unter Totraum soll in diesem Zusammenhang ein Raum verstanden werden, in welchem ein Fluid (z. B. Kondensat) während des normalen Betriebs der Adapteranordnung über längere Zeit verbleiben kann. In solchen Toträumen besteht die Gefahr, daß sich Bakterien festsetzen und auch eine Sterilisation überstehen.

Die erfindungsgemäße Verbindung ist bevorzugt anwendbar, wenn der Umgebungsdruck größer ist als der Druck im Inneren der Anordnung. Durch die Verbindung eines durch den O-Ring, die zwei Systemabschnitte und die Axialhalteeinrichtung gebildeten Raumes mit der Umgebung ist dieser Raum bei einer Sterilisierung zugänglich, so daß sich dort keine Bakterien ansiedeln können.

Bevorzugt ist der O-Ring radial vorgespannt. Hierdurch wird z. B. verhindert, daß sich der O-Ring vor dem Verbinden der Systemabschnitte löst oder während des Verbindens in eine ungeeignete Lage kommt.

Bei einem weiteren bevorzugten Ausführungsbeispiel hat der eine Systemabschnitt eine äußere Schulter, die als Anlage für einen Steg oder einen sonstigen vorstehenden Kranz des anderen Systemabschnitts dient. Die Fertigungsgenauigkeit der Teile ist bevorzugt so, daß der O-Ring bei Anlage von Schulter und Steg im idealen elastischen Bereich komprimiert ist.

Um im Innenraum der Anordnung günstige Strömungsverhältnisse zu schaffen, kann der eine Systemabschnitt zumindest in seinem dem anderen Systemabschnitt zugewandten Bereich denselben Innendurchmesser haben wie dieser. Der dem anderen Systemabschnitt nicht zugewandte Bereich des einen Systemabschnitts kann einen beliebigen Innendurchmesser haben. Der Übergang zwischen den Bereichen sollte sanft verlaufen. Weiterhin kann der Spalt zwischen den zwei Systemabschnitten möglichst klein gemacht werden, um die laminare Strömung im Inneren so wenig wie möglich zu stören.

Bei einer weiteren bevorzugten Ausführungsform ist der eine Systemabschnitt generell über dem anderen Systemabschnitt angeordnet. Mittel zum Vermeiden von Toträumen sind dann bevorzugt so ausgelegt, daß eine Flüssigkeit in bestehenden Spalten oder Räumen unter dem Einfluß der Schwerkraft nach unten abfließen kann. So kann der obere Systemabschnitt an seinem Steg umfänglich Einschnitte haben, die einen Abfluß aus dem innenliegenden Spalt zwischen den Systemabschnitten unterstützen und so ausgebildet sind, daß die Strömung im Innenraum der Anordnung möglichst wenig gestört wird. Dies kann z. B. dadurch erreicht werden, daß der Querschnitt der Einschnitte sich von innen nach außen verjüngt, vorzugsweise gemäß einer hyperbolischen oder parabolischen Beziehung. Der an dem unteren Systemabschnitt nach oben zur Anlage an der Schulter des oberen Systemabschnitts vorstehende Steg ist vorzugsweise auf etwa der Höhe des O-Rings mit radialen Durchbrechungen versehen, die ein Abfließen aus dem außenliegenden Raum zwischen den zwei Systemabschnitten und dem O-Ring gewährleistet. Die Durchbrechungsachse kann vorzugsweise auch geneigt sein, um diesen Vorgang weiter zu unterstützen.

Die Axialhalteeinrichtung wird vorzugsweise durch eine Überwurfmutter mit Sägezahngewinde ausgeführt. Das Sägezahngewinde hat eine solche Ausrichtung, daß ein radiales Wachsen der mit dem Sägezahn versehenen Elemente z. B. bei höheren Temperaturen aufgrund einer Sterilisation möglich ist, ohne dabei die Dichtcharakteristik, also die Lagebeziehung zwischen den Systemabschnitten zu verändern. Eine Federscheibe gewährleistet, daß sich die Systemabschnitte nie so weit voneinander abheben, daß die Dichtung passiert werden kann.

Die Federscheibe hat vorzugsweise einen derartigen Querschnitt, daß Kondensat an ihr schräg nach unten abfließen kann, und in der Überwurfmutter sind im Bereich der Auflagefläche Durchbrechungen im Bereich des radial äußersten Endes der Federscheibe vorgesehen, so daß das an der Federscheibe abfließende Kondensat aus dem Raum zwischen Überwurfmutter und Systemabschnitten abfließen kann.

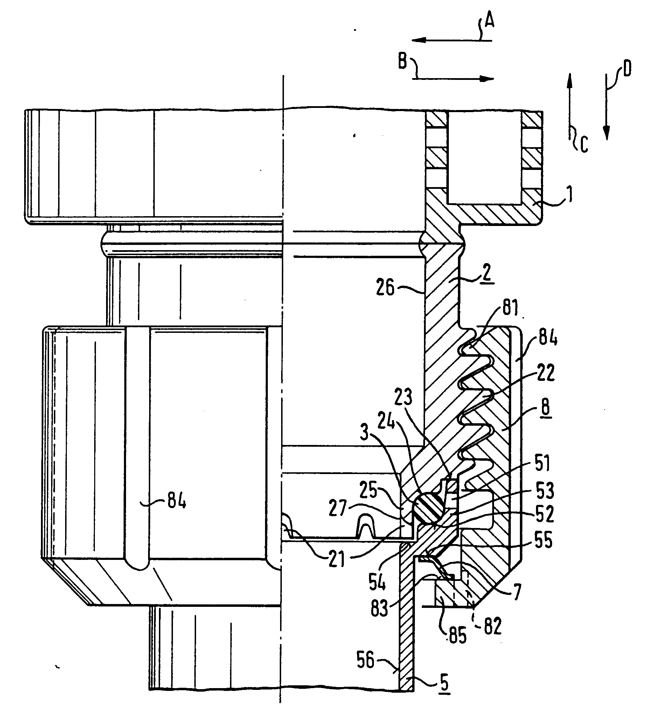

Weitere Vorteile, Merkmale und Anwendungsmöglichkeiten der vorliegenden Erfindung ergeben sich aus der nachfolgenden Beschreibung eines Ausführungsbeispieles in Verbindung mit der Zeichnung.

Die Figur zeigt eine Teil-Schnittansicht einer Ausführungsform des Adapters der Erfindung.

In der Figur sind gezeigt: ein Filterelement 1, ein Gewindeadapter 2 als ein Systemabschnitt, ein O-Ring 3, ein Gehäuseadapter 5 als ein anderer Systemabschnitt, eine Federscheibe 7 und eine Überwurfmutter 8.

Die Pfeile A, B, C und D dienen der Zuweisung von Richtungsangaben, wobei A nach innen und B nach außen zeigt (gilt nur für die rechte Hälfte der Figur). Die Pfeile C und D zeigen in Längsrichtung, wobei C nach oben und D nach unten zeigt.

Der Gewindeadapter 2 ist an dem Filterelement 1 angebracht, und zwar durch Schweißen, Kleben etc.. Der Gewindeadapter 2 hat einen oberen Abschnitt 26 mit großem Innendurchmesser und einen unteren Abschnitt 27 mit kleinerem Durchmesser. Der Innendurchmesser des unteren Abschnitts 27 entspricht dem Innendurchmesser des Gehäuseadapters 5. Der Übergang von dem oberen Abschnitt 26 zum unteren Abschnitt 27 verläuft schräg.

Der Gewindeadapter 2 hat ein Sägezahn-Außengewinde 22, dessen schräge Flächen von innen unten nach außen oben zeigen. Unterhalb des Außengewindes 22 ist etwas nach innen eingerückt eine nach außen offene Schulter 23 vorgesehen. Zwischen der Schulter 23 und dem unteren Ende des Gewindeadapters 2 ist eine nach außen halboffene Nut 24 zur Aufnahme des O-Rings vorgesehen. Ein innerer Steg 25 der Nut 24 ist umfänglich in Abständen mit Einschnitten 21 versehen, deren Form z. B. V-förmig sein kann und die in der Regel nach oben etwa bis zu dem O-Ring 3 reichen, mit diesem aber nicht in Kontakt stehen. Die Ausschnittform der Einschnitte 21 ist so, daß die innere Einschnittiefe größer ist als die äußere Einschnittiefe, so daß sich an der Innenwand des Abschnitts 27 keine Strömungsabrißkanten bilden.

Die Nut 24 ist in ihrer Kehle entsprechend der Form des O-Rings 3 ausgebildet, d. h. z. B. eine runde Kehle, deren Radius dem Radius des O-Rings 3 entspricht.

Der Außendurchmesser des Gewindeadapters 2 ist in dem Bereich, auf dem der O-Ring 3 sitzt, so bemessen, daß dieser auf dem Gewindeadapter 2 mit einer Vorspannung sitzt. Die Vorspannung liegt im Bereich zwischen einem Prozent und fünf Prozent, vorzugsweise etwa zwei Prozent.

Der Gehäuseadapter 5 hat, von unten nach oben betrachtet, zunächst einen geraden Abschnitt mit einer Innenfläche 56, dessen Innendurchmesser dem Innendurchmesser des Abschnitts 27 entspricht. Es folgt dann eine nach innen offene Schulter 54, deren Tiefe in Querrichtung etwas breiter ist als der Steg 25 des Gewindeadapters 2. An die Schulter 54 schließt sich nach einem kurzen, längsverlaufenden Abschnitt eine nach innen halboffene Nut 52 an, deren Kehle dem O-Ring 3 entspricht, ähnlich der Kehle der Nut 24. Ein Außensteg 53 bildet das obere Ende des Gehäuseadapters 5 und erstreckt sich in Längsrichtung auf die Schulter 23 zu. Die Schulter 23 bildet einen Anschlag für den Gehäuseadapter 5. Der Steg 53 ist quer oder schräg nach oben (von außen nach innen gesehen) mit Bohrungen 51 versehen, deren unterster Bereich auf Höhe der oberen Hälfte, vorzugsweise in der Nähe der Mitte des O-Rings 3 liegt und welche ein einwandfreies Ablaufen des Mediums (Fluid) unterstützen. An der Außenfläche des Gehäuseadapters 5 ist eine zur Längsmittelachse der Anordnung querverlaufende Gegenfläche 55 vorgesehen.

Die Überwurfmutter 8 hat ein Sägezahn-Innengewinde 81, das dem Außengewinde 22 des Gewindeadapters 2 entspricht, eine querverlaufende, innere Auflagefläche 83, die so angeordnet ist, daß die Federscheibe 7 Wirkung zwischen der Gegenfläche 55 und der Auflagefläche 83 entfalten kann, und ist außenumfänglich mit Längsnuten 84 versehen, an die ein geeigneter Schlüssel zum Drehen der Überwurfmutter 8 angesetzt werden kann. Dies bat gegenüber einer Sechskantmutter den Vorteil, daß das Anziehen der Mutter 8 unter beengten Verhältnissen auch zwischen mehreren Elementen ermöglicht wird. Innenumfänglich ist die Überwurfmutter 8 unterhalb des Sägezahn-Gewindes 81 so ausgebildet, daß sie den Gehäuseadapter 5 nicht berührt, damit der Gewinderaum leerlaufen kann. Ein Abschlußsteg 85 der Überwurfmutter 8 weist mehrere axiale Ablaufbohrungen 82, die so angeordnet sind, daß sie an der Federscheibe 7 vorbei eine Verbindung zwischen der Umgebung und dem Raum schaffen, der zwischen Überwurfmutter 8, Federscheibe 7, Gewindeadapter 2 und Gehäuseadapter 5 ausgebildet ist.

Beim Zusammenbau von zwei Systemabschnitten eines aseptischen Systems wird zunächst der O-Ring 3 unter radialer Vorspannung, wie zuvor beschrieben, in die Nut 24 eingesetzt. Die radiale Vorspannung verhindert, daß sich der O-Ring 3 z. B. unter dem Einfluß von Schwerkraft wieder von dem Gewindeadapter 2 löst. Hiernach wird der Gehäuseadapter 5 mit Überwurfmutter 8 und Federscheibe 7 von unten nach oben an den Gewindeadapter 2 angesetzt und mit diesem verschraubt. Die Überwurfmutter 8 wird bis zum Anschlag zwischen Schulter 23 und Steg 53 angezogen. Die Bauteile sind so präzise gefertigt, daß der O-Ring 3 bei Verschraubung bis zum Anschlag im idealen elastischen Bereich zwischen 10 % und 25 % Kompression arbeitet. Die Verschraubung gewährleistet, daß sich die Adapterelemente 2 und 5 nie so weit voneinander abheben, daß die Dichtung passiert werden kann. Die Federscheibe 7 trägt im wesentlichen dazu bei, daß sich die Verschraubung, die die Kompression gewährleistet, nicht löst. Die Ausbildung des Gewindes mit Sägezahnprofil gewährleistet, daß die Dichtcharakteristik konstant bleibt, auch wenn sich der Gewindeadapter 2, der in der Regel aus Kunststoff besteht, z. B. bei einem Temperaturanstieg während einer Sterilisation radial ausdehnt. Es ist klar, daß nicht die schrägverlaufenden, sondern die querverlaufenden Abschnitte des Sägezahnprofils für die axiale Haltewirkung verantwortlich sind.

Im Inneren der Adapteranordnung steht die Frage der Totraumfreiheit unter der Maßgabe, daß eine Fluidströmung im Inneren möglichst störungsfrei verlaufen soll. Zu diesem Zweckr ist der Gewindeadapter 2 so ausgebildet, daß dessen unterer Abschnitt 27 im Innendurchmesser dem Innendurchmesser des Gehäuseadapters 5 entspricht und im zusammengeschraubten Zustand der Adapteranordnung (bis zum Anschlag) nur ein kleiner Spalt zwischen den beiden Adapterelementen 2, 5 verbleibt. Um dennoch Toträume zu vermeiden, ist der Gewindeadapter 2 umfänglich am Steg 25 mit den V-förmigen Einschnitten 21 versehen. Kondensat, welches sich im Bereich des O-Rings 3 zwischen dem Steg 25 und der Schulter 54 gebildet hat, kann so über die Einschnitte 21 abfließen. Die Einschnitte 21 sind strömungsgünstig ausgebildet, wobei die Einschnittiefe in Längsrichtung innen größer ist als außen mit einem sanften Übergang.

Die Gefahr eines Totraumes in dem Bereich zwischen O-Ring 3, Gewindeadaper 2 und Gehäusadapter 5 wird dadurch ausgeschaltet, daß der Gehäusadapter 5 in seinem Steg 53 mit Ablaufbohrungen 51 versehen ist, die so angeordnet sind, daß ein Fluid (z. B. Kondensat) aus dem genannten Bereich abfließen kann. Wie aus der Zeichnung zu ersehen ist, ist die Anordnung der Adapterkomponenten in diesem genannten Bereich so, daß bei stehender Anordnung (Gewindeadapter über Gehäuseadapter) das Kondensat allein unter dem Einfluß der Schwerkraft abfließen kann.

Im Raum, der zwischen Gewindeadapter 2, Gehäuseadapter 5, Federscheibe 7 und Überwurfmutter 8, gebildet wird, wird Totraumfreiheit erzielt durch die Ablaufbohrungen 82 in der Überwurfmutter 8, wobei auch hier die Ausführung aller Adapterkomponenten so ist, daß ein Fluidpunkt in diesem Raum allein unter dem Einfluß der Schwerkraft abfließen kann (so ist die Federscheibe 7 z.B. derart ausgebildet, daß das Fluid schräg an ihr ablaufen kann).

### Bezugszeichenliste

- 1: Filterelement
- 2: Gewindeadapter
- 3: O-Ring
- 5: Gehäuseadapter
- 7: Federscheibe
- 8: Überwurfmutter

- 21: Einschnitte
- 22: Sägezahn-Außengewinde
- 23: Schulter
- 24: Nut
- 25: Steg
- 26: oberer Abschnitt
- 27: unterer Abschnitt

- 51: Ablaufbohrung
- 52: Nut
- 53: Steg
- 54: Schulter
- 55: Gegenfläche
- 56: Innenfläche

- 81: Sägezahn-Innengewinde
- 82: Ablaufbohrung
- 83: Auflagefläche
- 84: Längsnuten
- 85: Abschlußsteg

## Patentansprüche

1. Verbindung von zwei Fluid- Systemabschnitten (2,5), vorzugsweise eines aseptischen Systems, welche aufweist:
einen ersten Systemabschnitt (2), der einen O-Ring (3) in einer radial nach außen offenen Aussparung (24) trägt, einen zweiten Systemabschnitt (5), eine Axialhalteeinrichtung (22,7,8) und einen Raum zwischen dem O-Ring (3), den beiden Systemabschnitten (2,5) und der Axialhalteeinrichtung (22,7,8)
dadurch gekennzeichnet,
daß die Aussparung (24) zum zweiten Systemabschnitt hin öffnet, daß der zweite Systemabschnitt durch die Axialhalteeinrichtung (22,7,8) gegen den O-Ring (3) gedrückt wird, und daß der Raum zur Umgebung offen ist.

2. Verbindung nach Anspruch 1, wobei der O-Ring (3) radial um 1 bis 5 %, vorzugsweise um etwa 2 % elastisch vorgespannt ist.

3. Verbindung nach Anspruch 1 oder 2, wobei der zweite Systemabschnitt (5) eine zum ersten Systemabschnitt (2) und radial nach innen offene Aussparung (52) zur Aufnahme und Anlage an dem O-Ring (3) aufweist, der äußere Steg der Aussparung (52) länger ist als der Durchmesser des O-Rings (3) und der erste Systemabschnitt (2) eine äußere Radialschulter (23) aufweist, die als Anlage für den äußeren Steg (53) dient, wobei der O-Ring (3) im idealen elastischen Bereich komprimiert ist.

4. Verbindung nach einem der Ansprüche 1 bis 3, wobei die Innenfläche (27) des inneren Steges (25) der Aussparung (24) mit der Innenfläche (56) des zweiten Systemabschnittes (5) fluchtet.

5. Verbindung nach Anspruch 4, wobei der erste Systemabschnitt (2) über dem zweiten Systemabschnitt (5) liegt, der innere Steg (25) mindestens einen Einschnitt (21) hat, und der äußere Steg (53) mindestens eine etwa radial verlaufende Durchbrechung (51) hat.

6. Verbindung nach einem der Ansprüche 1 bis 5, wobei die Axialhalteeinrichtung (22,7,8) ein Sägezahn-Außengewinde (22) an einem Systemabschnitt (2), eine Überwurfmutter (8) mit Sägezahn-Innengewinde (81) und eine Federscheibe (7) umfaßt, die an einer Auflagefläche (83) der Überwurfmutter (8) und einer Gegenfläche (55) des anderen Systemabschnittes (5) angreift.

7. Verbindung nach Anspruch 6, wobei die Überwurfmutter (8) im Bereich der Auflagefläche (83) mindestens eine etwa axial verlaufende Durchbrechung (82) hat.

## Claims

1. Connector for two parts (2,5) of a fluid system, preferably an aseptic system, which comprises:
a first system part (2) carrying an O-ring (3) in a groove (4), which opens radially outwardly, a second system part (5), an axial holding device (22,7,8) and a space between the O-ring (3), two system parts (2,5) and the axial holding device (22,7,8),
characterized in that
the groove (24) opens to the second system part, the second system part is pressed against the O-ring (3) by the axial holding device, and said space is open to the surroundings.

2. Connector of claim 1, wherein the O-ring (3) is elastically biased by an amount of 1 to 5 %, preferably about 2 %.

3. Connector of claim 1 or 2, wherein the second system part (5) comprises a groove (52), which opens to the first system part (2) and which opens radially inwardly for accepting and abutting the O-ring (3), the outer ridge portion (53) of the groove (52) is longer than the diameter of the O-ring (3) and the first system part (2) comprises an outer radial shoulder (23) which serves as an abutment for the outer ridge portion (53), where the O-ring (3) is compressed within the ideal elastic range.

4. Connector of one of the preceding claims 1 to 3 wherein the inner surface (27) of an inner ridge portion (25) of the groove (24) is aligned flush with the inner surface (56) of the second system part (5).

5. Connector of claim 4, wherein the first system part (2) lies above the second system part (5), the inner ridge portion (25) comprises at least one recess (21) and the outer ridge portion (53) comprises at least one bore (51) running approximately radially.

6. Connector of one of the preceding claims 1 to 5, wherein the axial holding device (22,7,8) comprises a saw-tooth outer threading (22) on the first system part (2), a union nut (8) with a saw-tooth inner threading (81) and a spring washer 7, which engages with an abutment surface (83) and a countersurface (55) of the second system part (5).

7. Connector of claim 6, wherein the union nut (8) comprises at least one approximately axial hole (82) in the region of the abutment surface (83).

## Revendications

1. Assemblage de deux parties (2, 5) d'un système fluidique, de préférence un système aseptique, présentant :
une première partie de système (2), qui porte un joint torique (3) dans un évidement (24) ouvert radialement vers l'extérieur, une seconde partie de système (5), un dispositif de retenue axiale (22, 7, 8) et un espace entre le joint torique (3), les deux parties de système (2, 5) et le dispositif de retenue axiale (22, 7, 8),
**caractérisé** en ce que l'évidement (24) s'ouvre vers la seconde partie de système, en ce que la seconde partie de système est pressée contre le joint torique (3) par le dispositif de retenue axiale (22, 7, 8), et en ce que l'espace est ouvert vers l'environnement.

2. Assemblage selon la revendication 1, **caractérisé** en ce que le joint torique (3) est élastiquement précontraint en direction radiale de 1 à 5 %, de préférence d'environ 2 %.

3. Assemblage selon la revendication 1 ou 2, **caractérisé** en ce que la seconde partie de système (5) présente un évidement (52), qui est ouvert vers la première partie de système (2) et radialement vers l'intérieur et qui est destiné à la réception du joint torique (3) et à l'application contre ce dernier, la longueur de la bordure extérieure de l'évidement (52) est supérieure au diamètre du joint torique (3), et la première partie de système (2) présente un épaulement radial extérieur (23) qui sert d'appui pour la bordure extérieure (53), le joint torique (3) étant comprimé dans la plage idéalement élastique.

4. Assemblage selon l'une quelconque des revendications 1 à 3, **caractérisé** en ce que la face intérieure (27) de la bordure intérieure (25) de l'évidement (24) est à fleur de la face intérieure (56) de la seconde partie de système (5).

5. Assemblage selon la revendication 4, **caractérisé** en ce que la première partie de système (2) se trouve au-dessus de la seconde partie de système (5), la bordure intérieure (25) possède au moins une entaille (21), et la bordure extérieure (53) possède au moins une découpure (51) s'étendant approximativement radialement.

6. Assemblage selon l'une quelconque des revendications 1 à 5, **caractérisé** en ce que le dispositif de retenue axiale (22, 7, 8) comprend un filetage extérieur en dents de scie (22) sur une partie de système (2), un écrou-raccord (8) à filetage intérieur en dents de scie (81) et une rondelle-ressort (7), qui agit contre une face d'appui (83) de l'écrou-raccord (8) et une contre-surface (55) de l'autre partie de système (5).

7. Assemblage selon la revendication 6, **caractérisé** en ce que l'écrou-raccord (8) possède, dans la région de la face d'appui (83), au moins une découpure (82) s'étendant approximativement axialement.
